# EUROPEAN PATENT SPECIFICATION

(11) **EP 3 432 806 B1**
(45) Date of publication and mention of the grant of the patent: **02.10.2019**
(21) Application number: 17758020.6
(22) Date of filing: 17.08.2017
(51) Int. Cl.: A61B 17/04, A61F 2/24

(54) **TISSUE ANCHORS WITH FLEXIBLE TIPS FOR INSERTION INTO THE PERICARDIAL CAVITY**
GEWEBEANKER MIT FLEXIBLEN SPITZEN ZUM EINSETZEN IN DIE PERIKARDHÖHLE
ANCRAGES TISSULAIRES À POINTES FLEXIBLES POUR INSERTION DANS LA CAVITÉ PÉRICARDIQUE

(30) Priority: 18.08.2016 US 201662376685 P; 10.07.2017 WO PCT/IL2017/050771
(43) Date of publication of application: 30.01.2019
(73) Proprietor: 4Tech Inc., Waltham, MA 02452-8496 (US)
(72) Inventor: TOBIS, Idan, 99789 Beth Hashmonai (IL); LABORDE, Jean Claude, Nottingham Nottinghamshire NG12 5NB (GB); SOBRINO-SERRANO, Gabriel, Kinvara County Galway H91W2KX (IE); GRIFFIN, Patrick, Castlegar County Galway (IE)
(74) Representative: Evens, Paul Jonathan
(86) International application number: PCT/US2017/047442
(87) International publication number: WO 2018/035378

(56) References cited:
- WO-A1-2016/087934
- US-A1- 2002 013 571

## Description

### FIELD OF THE APPLICATION

The present invention relates generally to tissue anchors, and specifically to tissue anchors for implantation in soft tissue, such as cardiac tissue.

### BACKGROUND OF THE APPLICATION

Tissue anchors are used for anchoring elements, such as pacemaker electrode leads or sutures, to tissue, such as bone or soft tissue. PCT Publication WO 2016/087934 to Gilmore et al., describes an expandable tissue anchor according to the preamble of appended independent claim 1, that includes a shaft, a tissue-coupling element, and a flexible elongate tension member. The tissue-coupling element includes a wire, which is shaped as an open loop coil having, in some applications, more than one coil revolution when the tissue anchor is unconstrained, i.e., expanded from a linear state to a coiled state. The tension member includes a distal portion, that is fixed to a site on the open loop coil, a proximal portion, which has a longitudinal segment that runs alongside at least a portion of the shaft, and a crossing portion, which (i) is disposed between the distal and the proximal portions along the tension member, and (ii) crosses at least a portion of the open loop when the tissue anchor is expanded. The tissue anchor is configured to allow relative axial motion between the at least a portion of the shaft and the longitudinal segment of the proximal portion of the tension member when the tissue anchor is expanded.

US Patent Application Publication 2002/0013571 to Goldfarb et al. describes methods and devices for grasping, and optional repositioning and fixation of the valve leaflets to treat cardiac valve regurgitation, particularly mitral valve regurgitation.

### SUMMARY OF THE APPLICATION

In accordance with the present invention, there is provided an expandable tissue anchor as defined in appended independent claim 1. Embodiments of the invention are defined in appended claims which depend on independent claim 1.

Embodiments of the present invention provide an expandable tissue anchor that is deliverable to a cardiac chamber in an unexpanded generally elongate configuration within a deployment tool. The expandable tissue anchor is configured to be anchored to a cardiac tissue wall at a target site such that a tensile force can be applied to the expandable tissue anchor and thus to the cardiac tissue wall, once the expandable tissue anchor is deployed, so as to move the cardiac tissue wall at the target site relative to adjacent cardiac tissue. For some applications, such motion alters the geometry of a cardiac valve, such as the tricuspid valve or the mitral valve.

An expandable tissue anchor is provided configured to be deliverable to a cardiac chamber in an unexpanded generally elongate configuration within a percutaneous deployment tool, where the expandable tissue anchor is configured to be anchored to a cardiac tissue wall at a target site such that a tensile force can be applied to the expandable tissue anchor and thus to the cardiac tissue wall, once the expandable tissue anchor is deployed, so as to move the cardiac tissue wall at the target site relative to adjacent cardiac tissue. In some applications, the expandable tissue anchor comprises (1) an elongate tissue-coupling portion supported by an anchor shaft at a first end of the tissue-coupling portion, where the tissue-coupling portion is configured to be delivered in an unexpanded generally elongate configuration through the cardiac tissue wall from a first side of the wall to a second side of the wall, the tissue-coupling portion is further configured to expand, on the second side of the cardiac tissue wall, to an expanded open shape coil configuration generally orthogonal to the anchor shaft, such that the expanded tissue-coupling portion can be drawn tightly against the second side of the cardiac tissue wall at the target site when the tensile force is applied to the tissue-coupling portion, and where the tissue-coupling portion having a first stiffness, and (2) an elongate tip portion supported at a second end of the tissue-coupling portion and configured to be delivered through the cardiac tissue wall ahead of the tissue-coupling portion, where the tip portion has a second stiffness less than the first stiffness.

In some applications, the tip portion is configured such that when the cardiac tissue wall is a myocardial tissue wall, the tip portion can be directed, upon deployment in the pericardial cavity on the second side of the myocardial tissue wall, into the pericardial cavity ahead of the tissue-coupling portion, generally alongside and against pericardial tissue surrounding the myocardial tissue wall, without penetrating the pericardial tissue.

The tip portion may be axially fixed with respect to the tissue-coupling portion, or may be integral to the tissue-coupling portion. In some applications, the tip portion comprises a core wire and a coil wire wound around the core wire. In some applications, the tissue-coupling portion comprises a proximal portion and a distal bullet head, which is fixed to a distal end of the proximal portion, and has, at a widest longitudinal site along the bullet head, a greatest outer cross-sectional area that equals at least 150% of an average outer cross-sectional area of the proximal portion, and the bullet head serves as a crimp to secure the coil wire of the tip portion to the tissue-coupling portion.

In some applications, the tissue-coupling portion and the tip portion comprise a hollow tube. In some applications, the first stiffness of the tissue-coupling portion is at least 10% greater than the second stiffness of the tip portion. In some applications, the tip portion has a length of at least 3 mm. In some applications, the tip portion is not axially compressible.

In some applications, the tip portion comprises a plurality of slits that provides the lower stiffness than the tissue-coupling portion. In some cases, an average outer diameter of the tip portion is less than an average outer diameter of the tissue-coupling portion so as to provide the lower stiffness of the tip portion.

In some applications, the tip portion comprises a core wire and a coil wire wound around the core wire. In some applications, the tissue-coupling portion comprises a proximal portion and a distal bullet head, which is fixed to a distal end of the proximal portion, and has, at a widest longitudinal site along the bullet head, a greatest outer cross-sectional area that equals at least 150% of an average outer cross-sectional area of the proximal portion, and the bullet head serves as a crimp to secure the tip portion to the tissue-coupling portion.

In some applications, the expandable tissue anchor further comprises an elongate tension member coupled to a portion of the tissue-engaging coupling portion such that the tensile force can be applied to the tissue-coupling portion after it has been expanded to the open shape configuration. In some applications, the tip portion is an integral distal extension of the elongate tension member. In some applications, the tip portion is shaped as a closed loop, with a distal end of the elongate tension member fixed to the tissue-coupling portion near the second end of the tissue-coupling portion. In some applications, the tissue-coupling portion comprises a proximal portion and a distal bullet head, which is fixed to a distal end of the proximal portion, and has, at a widest longitudinal site along the bullet head, a greatest outer cross-sectional area that equals at least 150% of an average outer cross-sectional area of the proximal portion, and the bullet head serves as a crimp to secure the distal end of the elongate tension member to the tissue-coupling portion.

In some applications, an anchor system is provided that comprises the tension member expandable tissue anchor applications contemplated herein, and further comprises a tether affixed to the elongate tension member such that the tensile force can be applied to the expandable tissue anchor via the tether and the elongate tension member. In some applications, the anchor system comprises a second expandable tissue anchor, separate and distinct from the first expandable tissue anchor.

There is further provided a method for moving a cardiac tissue wall at a target site relative to adjacent cardiac tissue, the method including:
delivering, to a cardiac chamber, an expandable tissue anchor in an unexpanded generally elongate configuration within a deployment tool, the expandable tissue anchor including (a) an elongate tissue-coupling portion supported by an anchor shaft at a first end of the tissue-coupling portion, and including material having a first stiffness and (b) an elongate tip portion supported at a second end of the tissue-coupling portion, and including material having a second stiffness less than the first stiffness;
delivering the elongate tip portion through the cardiac tissue wall ahead of the tissue-coupling portion;
delivering the tissue-coupling portion in an unexpanded generally elongate configuration through the cardiac tissue wall from a first side of the wall to a second side of the wall, such that the tissue-coupling portion expands, on the second side of the cardiac tissue wall, to an expanded open shape configuration, thereby anchoring the expandable tissue anchor to the cardiac tissue wall at the target site; and
tightly drawing the expanded tissue-coupling portion against the second side of the cardiac tissue wall at the target site by applying a tensile force to the tissue-coupling portion, and thus to the cardiac tissue wall, so as to move the cardiac tissue wall at the target site relative to the adjacent cardiac tissue.

For some applications, the cardiac tissue wall is a myocardial tissue wall, and delivering the tip portion through the cardiac tissue wall ahead of the tissue-coupling portion includes directing the tip portion, upon deployment in the pericardial cavity on the second side of the myocardial tissue wall, into the pericardial cavity ahead of the tissue-coupling portion, generally alongside and against pericardial tissue surrounding the myocardial tissue wall, without penetrating the pericardial tissue.

For some applications, applying a tensile force to the tissue-coupling portion includes applying the tensile force to an elongate tension member coupled to a portion of the tissue-coupling portion. For some applications, applying the tensile force to the elongate tension member includes applying the tensile force to a tether affixed to the elongate tension member.

For some applications, the method further includes implanting a second expandable tissue anchor, separate and distinct from the expandable tissue anchor.

The present invention will be more fully understood from the following detailed description of embodiments thereof, taken together with the drawings, in which:

### BRIEF DESCRIPTION OF THE DRAWINGS

Fig. 1 is a schematic illustration of an expandable tissue anchor, in accordance with an application of the present invention;
Figs. 2A-B are schematic illustrations of additional configurations of another expandable tissue anchor, in accordance with respective applications of the present invention;
Fig. 3 is a schematic illustration of yet another configuration of yet another expandable tissue anchor, in accordance with an application of the present invention;
Fig. 4 is a schematic illustration of still another configuration of still another expandable tissue anchor, in accordance with an application of the present invention;
Fig. 5 is a schematic illustration of an additional configuration of additional expandable tissue anchor, in accordance with an application of the present invention; and
Figs. 6A-D are schematic illustrations of method of deploying the expandable tissue anchor of Fig. 1 through a myocardial tissue wall.

### DETAILED DESCRIPTION OF APPLICATIONS

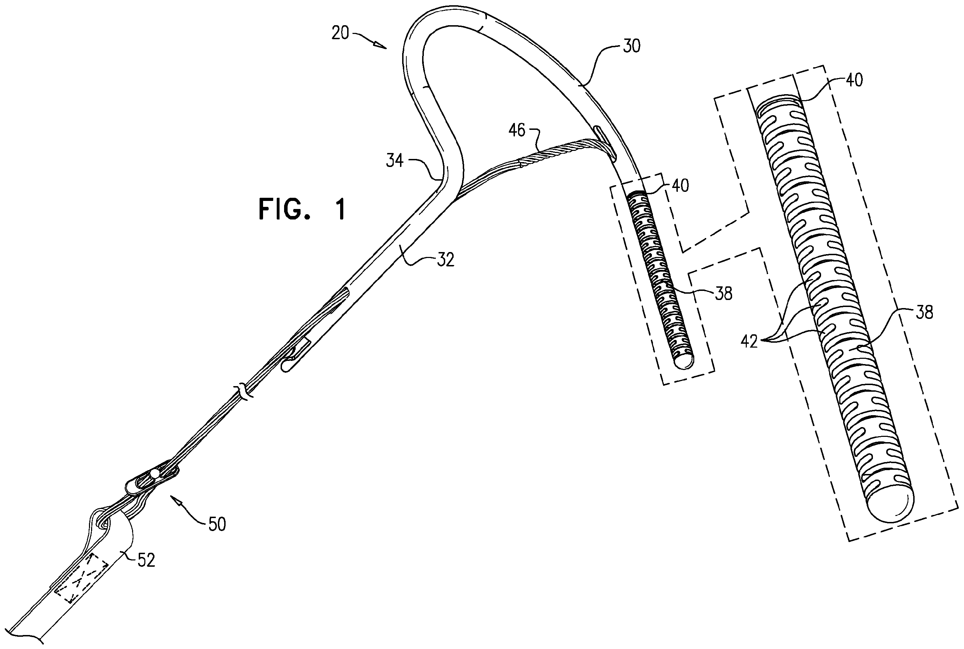

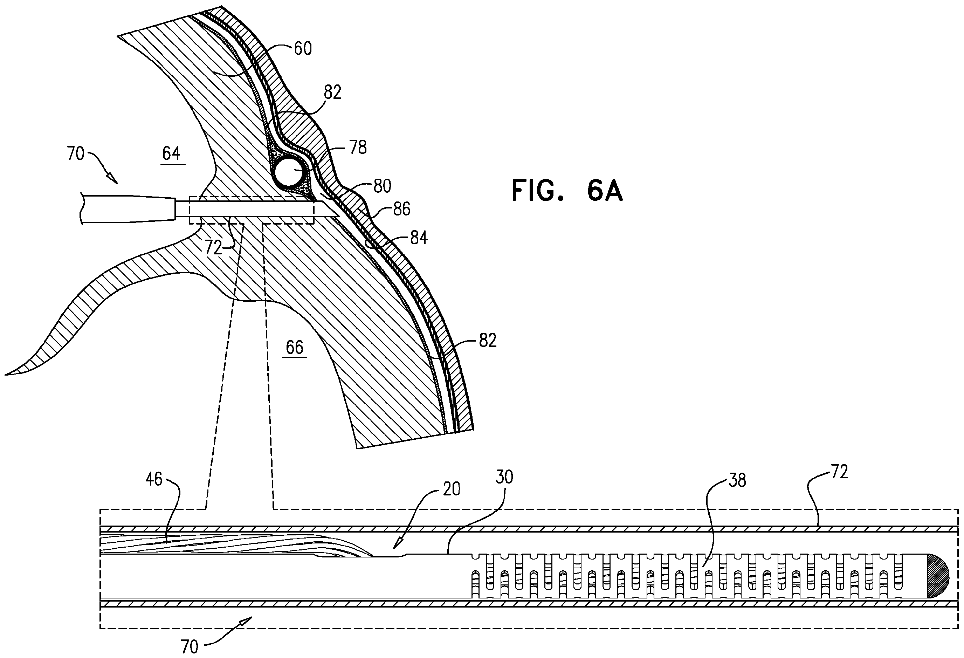

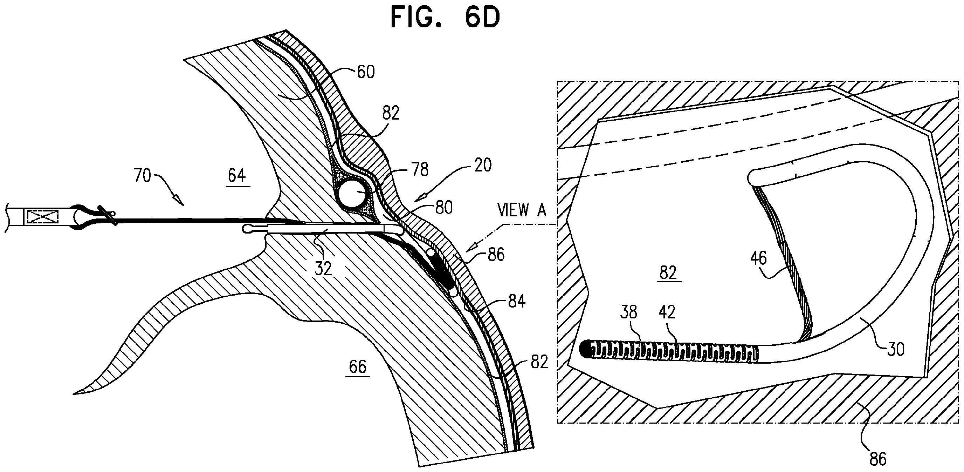

Referring to Fig. 1, an expandable tissue anchor 20 is provided that is configured to be anchored to a cardiac tissue wall at a target site such that a tensile force can be applied to expandable tissue anchor 20 and thus to the cardiac tissue wall, so as to move the cardiac tissue wall at the target site relative to adjacent cardiac tissue. Expandable tissue anchor 20 comprises an elongate tissue-coupling portion 30 supported by an anchor shaft 32 at a first end 34 of tissue-coupling portion 30. Tissue-coupling portion 30 is configured to be delivered in an unexpanded generally elongate configuration, such as described hereinbelow with reference to Fig. 6A, through the cardiac tissue wall from a first side of the wall to a second side of the wall, such as described hereinbelow with reference to Figs. 6A-D. Tissue-coupling portion 30 is further configured, upon deployment, to expand, on the second side of the cardiac tissue wall, to an expanded coil (open shape) configuration, such as described hereinbelow with reference to Fig. 6D, such that the expanded tissue-coupling portion 30 can be drawn tightly against the second side of the cardiac tissue wall at the target site when the tensile force is applied to tissue-coupling portion 30. Tissue-coupling portion 30 has a first stiffness.

Expandable tissue anchor 20 further comprises an elongate tip portion 38 supported at a second end 40 of tissue-coupling portion 30 and configured to be delivered through the cardiac tissue wall ahead of tissue-coupling portion 30, such as described hereinbelow with reference to Figs. 6A-D. Tip portion 38 has a second stiffness less than the first stiffness of the material of tissue-coupling portion 30. For example, the first stiffness of tissue-coupling portion 30 may be at least 10% greater than the second stiffness of tip portion 38, such as 20% or 30% greater.

Tissue-coupling portion 30, once expanded on the second side of the cardiac tissue wall, such as described hereinbelow with reference to Fig. 6D, comprises a configuration generally orthogonal to anchor shaft 32. For some applications, such as shown in Fig. 1, tip portion 38 is comprises a plurality of slits 42 that provides the lower stiffness of tip portion 38 than of tissue-coupling portion 30. For some applications, the tissue-coupling portion 30 and tip portion 38 comprise the same material, but they need not. Slits 42 may be oriented circumferentially around tip portion 38 (as shown); axially along tip portion 38 (configuration not shown); in another orientation, such as helically around tip portion 38 (configuration not shown); or in a plurality of different orientations. For some applications, such as shown in Fig. 1, tissue-coupling portion 30 and tip portion 38 are integral to one another, although they may also be discrete components fixed together. For some applications, tissue-coupling portion 30 and tip portion 38 comprise a hollow tube. In some applications, tip portion 38 is not axially compressible. For some applications, tip portion 38 has a length of at least 3 mm, such as at least 5 mm, or even at least 20 mm (but typically no more than 60 mm), which may additionally provide a clear indication of pericardial placement.

Referring to Fig. 1, for some applications, expandable tissue anchor 20 further comprises an elongate tension member 46 coupled to a portion of tissue-coupling portion 30. Through elongate tension member 46, or components equivalent thereto, a tensile force can be applied to tissue-coupling portion 30 after it has been expanded to the coil open shape configuration. When applied *in vivo,* the tensile force may have the benefit of bringing the anchor close to the tissue wall to which it is applied. For some applications, an anchor system 50 is provided that comprises expandable tissue anchor 20 and a tether 52 affixed to elongate tension member 46 such that the tensile force can be applied to expandable tissue anchor 20 via tether 52 and elongate tension member 46. For some applications, anchor system 50 further comprises a second expandable tissue anchor, separate and distinct from expandable tissue anchor 20, such as is shown in PCT Publication WO 2016/087934. For some applications, the second expandable tissue anchor, and additional anchors if so desired, is couplable or coupled to expandable tissue anchor 20 by one or more tethers that include tether 52.

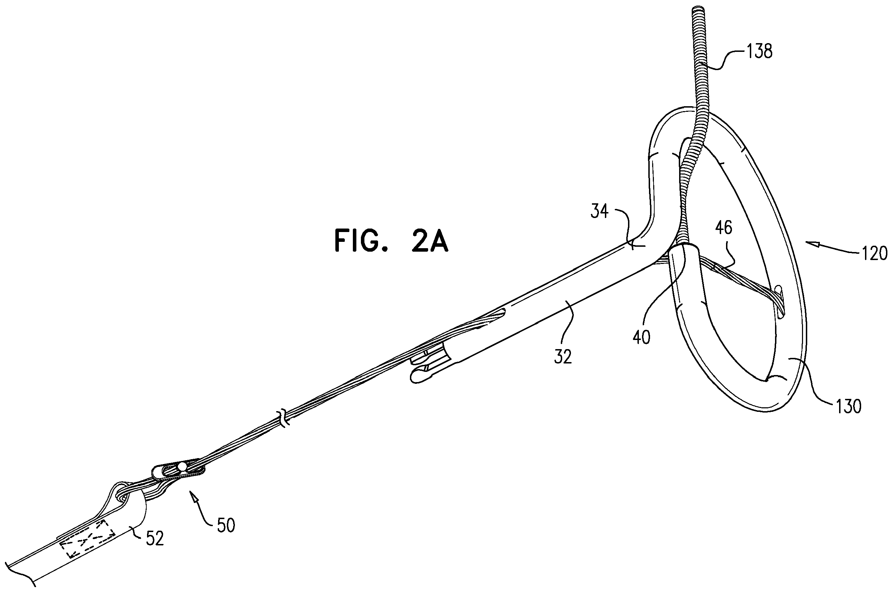

Referring to Fig. 2A, an expandable tissue anchor 120 is provided that is generally similar to expandable tissue anchor 20, except as described below. An elongate tip portion 138 of tissue anchor 120 may be made of the same or similar material as a tissue-coupling portion 130 of the tissue anchor, but the average outer diameter of tip portion 138 is less than an average outer diameter of tissue-coupling portion 130 so as to provide the lower stiffness of tip portion 138 than of tissue-coupling portion 130. The difference in stiffness properties may be implemented by a difference in material and/or a difference in dimensions. For example, the average outer diameter of tip portion 138 may be at least 10% less, such as at least 20% less, than the average outer diameter of tissue-coupling portion 130.

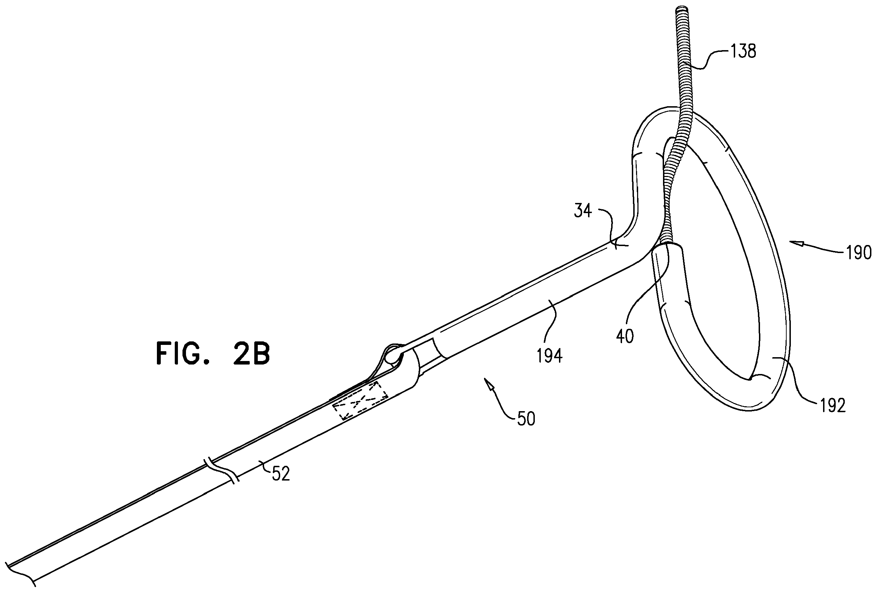

Reference is still made to Fig. 2A, and is additionally made to Fig. 2B, which is a schematic illustration of an expandable tissue anchor 190 that is generally similar to expandable tissue anchor 120, except as described below. Like tissue anchor 120, tissue anchor 190 comprises a tissue-coupling portion 192, elongate tip portion 138, and an anchor shaft 194 at first end 34 of tissue-coupling portion 192. However, unlike in the configuration of tissue anchor 120 shown in Fig. 2A, tissue anchor 190 does not comprise elongate tension member 46. Tension is instead applied to tissue-coupling portion 192 through anchor shaft 194, typically via tether 52. Although, for the sake of brevity, configurations of tissue anchors 20, 220, 320, and 420 are not illustrated without elongate tension member 46, it is to be understood that elongate tension member 46 is optional in all of the tissue anchors described herein, and that all of the tissue anchors described herein may be provided without elongate tension member 46, as shown in Fig. 2B for tissue anchor 190, *mutatis mutandis.*

Referring to Figs. 2A and 2B, for some applications, tissue-coupling portion 130 or 192 comprises a hollow tube, while for other applications, tissue-coupling portion 130 or 192 comprises a solid wire.

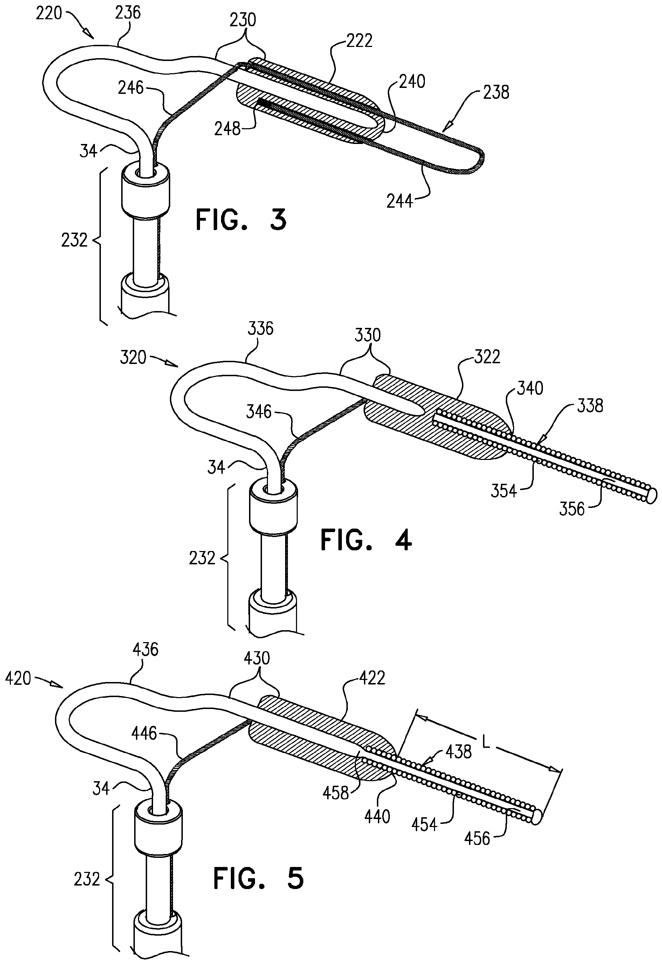

Referring to Fig. 3, an expandable tissue anchor 220 is provided that is generally similar to expandable tissue anchor 20, except as described below. An elongate tip portion 238 of tissue anchor 220 is provided as an integral distal extension of an elongate tension member 246, similar to elongate tension member 46 described hereinabove with reference to Fig. 1. In other words, a single elongate member (e.g., a solid wire or a hollow tube) defines both elongate tension member 246 and elongate tip portion 238. Elongate tip portion 238 is supported at, and extends distally beyond, a second end 240 of an elongate tissue-coupling portion 230. For some applications, as shown, elongate tip portion 238 is shaped as a closed loop 244, with a distal end 248 of elongate tension member 246 fixed to elongate tissue-coupling portion 230 near a second end 240 of the tissue-coupling portion. Typically, the average outer diameter of tip portion 238 is less than an average outer diameter of tissue-coupling portion 230 (e.g., at least 10% less, such as at least 20% less), so as to provide at least a portion of the lower stiffness of tip portion 238 than of tissue-coupling portion 230.

In some configurations, elongate tissue-coupling portion 230 comprises a narrow proximal portion 236 and a distal bullet head 222 (shown in cross section in Fig. 3), which is fixed to a distal end of proximal portion 236, and has, at a widest longitudinal site along bullet head 222, a greatest outer cross-sectional area that equals at least 150% (e.g., at least 200%, or at least 300%) of an average outer cross-sectional area of proximal portion 236. (The cross-sectional area of bullet head 222 is measured perpendicular to a central longitudinal axis of the bullet head. Similarly, the cross-sectional area of proximal portion 236 is measured perpendicular to a central longitudinal axis of proximal portion 236.) Typically, a distal end of distal bullet head 222 defines second end 240 of elongate tissue-coupling portion 230.

Bullet head 222 may increase the surface area of tissue-coupling portion 230 for pressing against myocardial tissue wall 60, described hereinbelow with reference to Figs. 6A-D, which may increase stability when force is applied to tissue-coupling portion 230. Bullet head 222 may also serve as a crimp to secure distal end 248 of elongate tension member 246 to elongate tissue-coupling portion 230. Typically, a portion of elongate tension member 246 passes through a channel defined through bullet head 222. Alternatively, the greatest outer cross-sectional area of bullet head 222 equals less than 150% of the average outer cross-sectional area of proximal portion 236, in which configuration bullet head 222 may serve only as a crimp for securing distal end 248 of elongate tension member 246 to elongate tissue-coupling portion 230.

Referring to Fig. 4, an expandable tissue anchor 320 is provided that is generally similar to expandable tissue anchor 120, except as described below. An elongate tip portion 338 of tissue anchor 320 is supported at a second end 340 of an elongate tissue-coupling portion 330 of tissue anchor 320, and may be made of the same or similar material as tissue-coupling portion 330, but the average outer diameter of a core wire 356 of elongate tip portion 338 is typically less than an average outer diameter of tissue-coupling portion 330 so as to provide the lower stiffness of tip portion 338 than of tissue-coupling portion 330. The difference in stiffness properties may be implemented by a difference in material and/or a difference in dimensions. For example, the average outer diameter of tip portion 338 may be at least 10% less, such as at least 20% less, than the average outer diameter of tissue-coupling portion 330.

For some applications, tip portion 338 comprises core wire 356 and a coil wire 354 wound around core wire 356. Core wire 356 may, for example, comprise Nitinol, and coil wire 354 may, for example, comprise a platinum-iridium alloy, and is typically radiopaque, such as for visualization under fluoroscopy.

In some configurations, elongate tissue-coupling portion 330 comprises a narrow proximal portion 336 and a distal bullet head 322 (shown in cross section in Fig. 4), which is fixed to a distal end of proximal portion 336, and has, at a widest longitudinal site along bullet head 322, a greatest outer cross-sectional area that equals at least 150% (e.g., at least 200%, or at least 300%) of an average outer cross-sectional area of proximal portion 336. (The cross-sectional area of bullet head 322 is measured perpendicular to a central longitudinal axis of the bullet head. Similarly, the cross-sectional area of proximal portion 336 is measured perpendicular to a central longitudinal axis of proximal portion 336.) A distal end of distal bullet head 322 defines second end 340 of elongate tissue-coupling portion 330.

Bullet head 322 may increase the surface area of tissue-coupling portion 330 for pressing against myocardial tissue wall 60, described hereinbelow with reference to Figs. 6A-D, which may increase stability when force is applied to tissue-coupling portion 330. Bullet head 322 also serves as a crimp to secure tip portion 338 to elongate tissue-coupling portion 330. Alternatively, the greatest outer cross-sectional area of bullet head 322 equals less than 150% of the average outer cross-sectional area of proximal portion 336, in which configuration bullet head 322 may serve only as a crimp for securing tip portion 338 to elongate tissue-coupling portion 330.

Optionally, tissue anchor 320 further comprises an elongate tension member 346, similar to elongate tension member 46 described hereinabove with reference to Fig. 1.

Referring to Fig. 5, an expandable tissue anchor 420 is provided that is generally similar to expandable tissue anchor 320, except as described below. An elongate tip portion 438 of tissue anchor 420 is supported at a second end 440 of an elongate tissue-coupling portion 430 of tissue anchor 420, and may be made of the same or similar material as tissue-coupling portion 430, but the average outer diameter of a core wire 456 of elongate tip portion 438 is typically less than an average outer diameter of tissue-coupling portion 430 so as to provide the lower stiffness of tip portion 438 than of tissue-coupling portion 430. The difference in stiffness properties may be implemented by a difference in material and/or a difference in dimensions. For example, the average outer diameter of tip portion 438 may be at least 10% less, such as at least 20% less, than the average outer diameter of tissue-coupling portion 430.

For some applications, elongate tissue-coupling portion 430 comprises a narrow proximal portion 436, and tip portion 438 comprises core wire 456 that is an integral distal extension of proximal portion 436 of tissue-coupling portion 430. Proximal portion 436 narrows at a tapering portion 458 to provide narrower core wire 456, as described above.

For some applications, tip portion 438 further comprises a coil wire 454 wound around core wire 456. Core wire 456 may, for example, comprise Nitinol, and coil wire 454 may, for example, comprise a platinum-iridium alloy, and is typically radiopaque.

In some configurations, elongate tissue-coupling portion 430 further comprises a distal bullet head 422 (shown in cross section in Fig. 5), which is fixed to a distal end of proximal portion 436, and has, at a widest longitudinal site along bullet head 422, a greatest outer cross-sectional area that equals at least 150% (e.g., at least 200%, or at least 300%) of an average outer cross-sectional area of proximal portion 436. (The cross-sectional area of bullet head 422 is measured perpendicular to a central longitudinal axis of the bullet head. Similarly, the cross-sectional area of proximal portion 436 is measured perpendicular to a central longitudinal axis of proximal portion 436.) A distal end of distal bullet head 422 defines second end 440 of elongate tissue-coupling portion 430.

Bullet head 422 may increase the surface area of tissue-coupling portion 430 for pressing against myocardial tissue wall 60, described hereinbelow with reference to Figs. 6A-D, which may increase stability when force is applied to tissue-coupling portion 430. Bullet head 422 may also serve as a crimp to secure coil wire 454 of tip portion 438 to elongate tissue-coupling portion 430. Alternatively, the greatest outer cross-sectional area of bullet head 422 equals less than 150% of the average outer cross-sectional area of wire 436, in which configuration bullet head 422 may serve only as a crimp for securing coil wire 454 to elongate tissue-coupling portion 430.

Optionally, tissue anchor 420 further comprises an elongate tension member 446, similar to elongate tension member 46 described hereinabove with reference to Fig. 1.

For some applications, tip portion 438 has a length L, distally beyond second end 440 of tissue-coupling portion 430, of at least 3 mm, such as at least 5 mm, or even at least 20 mm (but typically no more than 60 mm), which may additionally provide a clear indication of pericardial placement. (Tip portion 438 of tissue anchor 320, described hereinabove with reference to Fig. 4, may also have this length.)

Referring to Figs. 3-5, for some applications, narrow proximal portion 236, 336, or 436 comprises a solid wire, while for other applications, narrow proximal portion 236, 336, or 436 comprises a hollow tube.

Still referring to Figs. 3-5, for some applications, elongate tip portion 238, 338, or 438 is configured to at least partially assume a spiral shape when unconstrained in the pericardial cavity 80, such as described hereinbelow with reference to Figs. 6A-D.

Reference is now made to Figs. 6A-D, which are schematic illustrations of a method of deploying expandable tissue anchor 20 through a myocardial tissue wall 60. Although in Figs. 6A-D expandable tissue anchor 20 is shown deployed through a myocardial tissue wall, expandable tissue anchor 20 may also be deployed through other cardiac tissue walls, such as the interatrial septum, either at or not at the fossa ovalis, or through other non-cardiac tissue walls. Indeed, the tissue anchors described herein may be deployed in any number of bodily locations where it is desired to anchor into or behind tissue for purposes of moving such tissue relative to adjacent tissue. The method of Figs. 6A-D may also be used for deploying expandable tissue anchor 120, tissue anchor 190, tissue anchor 220, tissue anchor 320, and tissue anchor 420, described hereinabove with reference to Figs. 2A, 2B, 3, 4, and 5, respectively.

As shown in Fig. 6A, expandable tissue anchor 20 is delivered to a target site, such as a cardiac chamber, in an unexpanded generally elongate configuration within a deployment tool 70, which comprises a hollow needle 72. The cardiac chamber may be a right atrium 64 (as shown), a right ventricle 66 (configuration not shown), a left atrium (configuration not shown), or a left ventricle (configuration not shown). In one application, hollow needle 72 is used to puncture through a first side of a myocardial tissue wall 60 and visceral pericardium 82 (which is part of the epicardium), avoiding vasculature such as the right coronary artery (RCA) 78. The deployment tool 70 is then further directed into the pericardial cavity 80 between visceral pericardium 82 and parietal pericardium 84, carefully avoiding puncturing parietal pericardium 84 and fibrous pericardium 86.

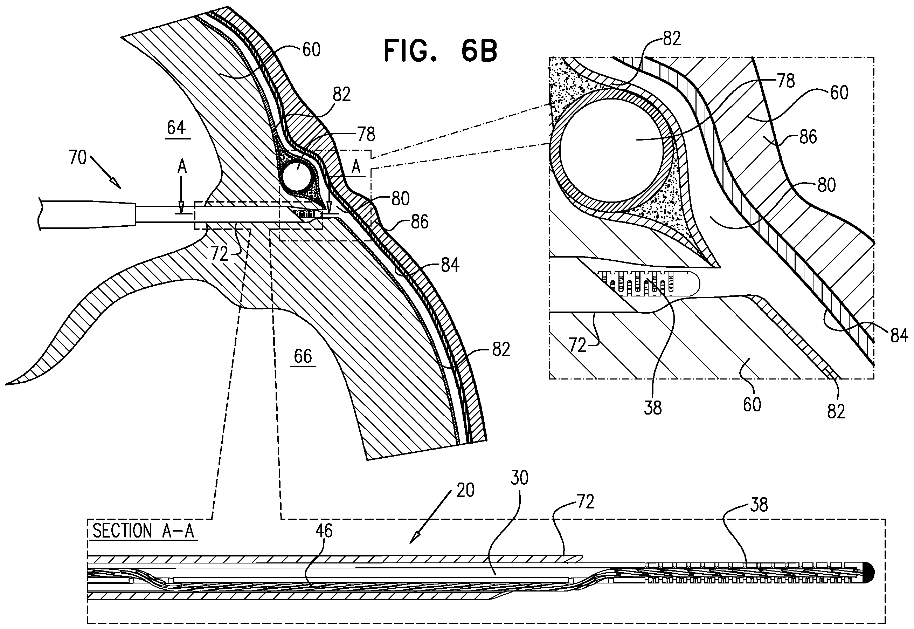

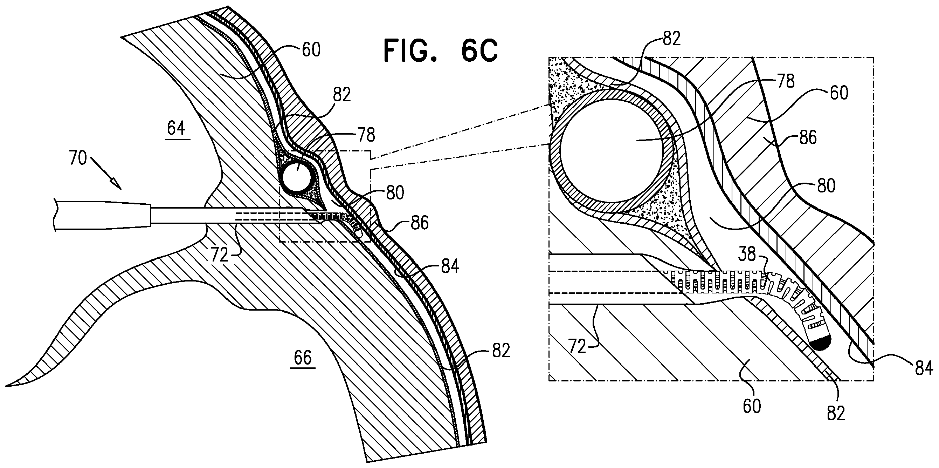

As shown in Fig. 6B, for some applications, hollow needle 72 is withdrawn slightly, exposing some or all of the tip portion of the expandable tissue anchor 20 within a bore created through myocardial tissue wall 60 by the hollow needle 72. As shown in Fig. 6C, elongate tip portion 38 of expandable tissue anchor 20 is delivered through myocardial tissue wall 60 ahead of tissue-coupling portion 30. Tip portion 38 can be directed, upon deployment in pericardial cavity 80 on the second side of myocardial tissue wall 60, into pericardial cavity 80 ahead of tissue-coupling portion 30, generally alongside and against pericardial tissue surrounding the myocardial tissue wall, without penetrating the pericardial tissue (particularly, parietal pericardium 84 and fibrous pericardium 86). Tip portion 38 is not stiff enough to inadvertently puncture the pericardial tissue.

As shown in Fig. 6D, tissue-coupling portion 30 of expandable tissue anchor 20 is delivered through myocardial tissue wall 60 and into pericardial cavity 80 following elongate tip portion 38. Elongate tip portion 38 directs tissue-coupling portion 30 generally alongside and against the pericardial tissue, thereby preventing the stiffer tissue-coupling portion 30 from puncturing the pericardial tissue (particularly, parietal pericardium 84 and fibrous pericardium 86). Tissue-coupling portion 30 expands to the coil open shape configuration on the second site of myocardial tissue wall 60, thereby anchoring expandable tissue anchor 20 to myocardial tissue wall 60.

Once expandable tissue anchor 20 has been anchored to myocardial tissue wall 60 at the target site, a tensile force is applied using tether 52 to expandable tissue anchor 20 and thus to myocardial tissue wall 60, so as to move myocardial tissue wall 60 at the target site relative to adjacent cardiac tissue. For some applications, such motion can have the benefit of altering the geometry of a nearby cardiac valve, such as the tricuspid valve or the mitral valve.

The following applications, which are assigned to the assignee of the present patent, disclose techniques that may be combined with embodiments of the present invention: US Patent 8,475,525 to Maisano et al.; US Patent 8,961,596 to Maisano et al.; US Patent 8,961,594 to Maisano et al.; PCT Publication WO 2011/089601; US Patent 9,241,702 to Maisano et al.; PCT Publication WO 2013/011502; US Provisional Application 61/750,427, filed January 9, 2013; US Provisional Application 61/783,224, filed March 14, 2013; PCT Publication WO 2013/179295; US Provisional Application 61/897,491, filed October 30, 2013; US Provisional Application 61/897,509, filed October 30, 2013; US Patent 9,307,980 to Gilmore et al.; PCT Publication WO 2014/108903; PCT Publication WO 2014/141239; US Provisional Application 62/014,397, filed June 19, 2014; PCT Publication WO 2015/063580; US Patent Application Publication 2015/0119936; US Provisional Application 62/086,269, filed December 2, 2014; US Provisional Application 62/131,636, filed March 11, 2015; US Provisional Application 62/167,660, filed May 28, 2015; PCT Publication WO 2015/193728; PCT Publication WO 2016/087934; US Patent Application Publication 2016/0242762; PCT Publication WO 2016/189391; and US Patent Application Publication 2016/0262741.

It will be appreciated by persons skilled in the art that the present invention is not limited to what has been particularly shown and described hereinabove. Rather, the scope of the present invention is defined by the claims, and may include both combinations and subcombinations of the various features described hereinabove, as well as variations and modifications thereof that are not in the prior art, which would occur to persons skilled in the art upon reading the foregoing description.

## Claims

1. An expandable tissue anchor (20, 120, 190, 220, 320, 420) configured to be delivered to a cardiac chamber in an unexpanded generally elongate configuration within a deployment tool (70), the expandable tissue anchor (20, 120, 190, 220, 320, 420) configured to be anchored to a cardiac tissue wall at a target site such that a tensile force can be applied to the expandable tissue anchor (20, 120, 190, 220, 320, 420) and thus to the cardiac tissue wall, once the expandable tissue anchor (20, 120, 190, 220, 320, 420) is deployed, so as to move the cardiac tissue wall at the target site relative to adjacent cardiac tissue, the expandable tissue anchor (20, 120, 190, 220, 320, 420) comprising:
an elongate tissue-coupling portion (30, 130, 192, 230, 330, 430) supported by an anchor shaft (32, 194) at a first end (34) of the tissue-coupling portion (30, 130, 192, 230, 330, 430), the tissue-coupling portion (30, 130, 192, 230, 330, 430) configured to be delivered in an unexpanded generally elongate configuration through the cardiac tissue wall from a first side of the wall to a second side of the wall, the tissue-coupling portion (30, 130, 192, 230, 330, 430) further configured to expand, on the second side of the cardiac tissue wall, to an expanded coil open shape configuration generally orthogonal to the anchor shaft (32, 194), such that the expanded tissue-coupling portion (30, 130, 192, 230, 330, 430) can be drawn tightly against the second side of the cardiac tissue wall at the target site when the tensile force is applied to the tissue-coupling portion (30, 130, 192, 230, 330, 430), the tissue-coupling portion (30, 130, 192, 230, 330, 430) having a first stiffness;
**characterized by**
an elongate tip portion (38, 138, 238, 338, 438) supported at a second end (40, 240, 340, 440) of the tissue-coupling portion (30, 130, 192, 230, 330, 430) and configured to be delivered through the cardiac tissue wall ahead of the tissue-coupling portion (30, 130, 192, 230, 330, 430), the tip portion (38, 138, 238, 338, 438) having a second stiffness less than the first stiffness.

2. The expandable tissue anchor (20, 120, 190, 220, 320, 420) according to Claim 1, wherein the tip portion (38, 138, 238, 338, 438) is axially fixed with respect to the tissue-coupling portion (30, 130, 192, 230, 330, 430).

3. The expandable tissue anchor (20, 120, 190, 320, 420) according to Claim 1, wherein the tip portion (38, 138, 338, 438) is not axially compressible.

4. The expandable tissue anchor (20, 120, 190, 220, 320, 420) according to Claim 1, wherein the first stiffness of the tissue-coupling portion (30, 130, 192, 230, 330, 430) is at least 10% greater than the second stiffness of the tip portion (38, 138, 238, 338, 438).

5. The expandable tissue anchor (20, 120, 190, 220, 320, 420) according to Claim 1, wherein the tip portion (38, 138, 238, 338, 438) has a length of at least 3 mm.

6. The expandable tissue anchor (20) according to Claim 1, wherein the tip portion (38) comprises a plurality of slits (42) that provides the lower second stiffness of the tip portion (38).

7. The expandable tissue anchor (120, 190) according to Claim 1, wherein an average outer diameter of the tip portion (138) is less than an average outer diameter of the tissue-coupling portion (130, 192) so as to provide the lower second stiffness of the tip portion (138).

8. The expandable tissue anchor (20, 120, 190, 220, 320, 420) according to Claim 1, wherein the tip portion (38, 138, 238, 338, 438) comprises a material different from that of the tissue-coupling portion (30, 130, 192, 230, 330, 430) so as to provide the lower second stiffness of the tip portion (38, 138, 238, 338, 438) relative to the first stiffness of the tissue-coupling portion (30, 130, 192, 230, 330, 430).

9. The expandable tissue anchor (20, 420) according to any one of Claims 1-8, wherein the tissue-coupling portion (30, 430) and the tip portion (38, 438) are integral to one another.

10. The expandable tissue anchor (20) according to Claim 9, wherein the tissue-coupling portion (30) and the tip portion (38) comprise a hollow tube.

11. The expandable tissue anchor (320, 420) according to any one of Claims 1-5 or 8-9, wherein the tip portion (338, 438) comprises a core wire (356, 456) and a coil wire (354, 454) wound around the core wire (356, 456).

12. The expandable tissue anchor (20, 120, 220, 320, 420) according to any one of Claims 1-11, further comprising an elongate tension member (46, 246, 346, 446) coupled to a portion of the tissue-coupling portion (30, 130, 230, 330, 430) such that the tensile force can be applied to the tissue-coupling portion (30, 130, 230, 330, 430) after it has been expanded to the coil open shape configuration.

13. The expandable tissue anchor (220) according to Claim 12,
wherein the tip portion (238) is an integral distal extension of the elongate tension member (246), and
wherein the tip portion (238) is shaped as a closed loop (244), with a distal end (248) of the elongate tension member (246) fixed to the tissue-coupling portion (230) near the second end (240) of the tissue-coupling portion (230).

14. An anchor system (50) comprising the expandable tissue anchor (20, 120, 220, 320, 420) according to Claim 12, wherein the anchor system (50) further comprises a tether (52) affixed to the elongate tension member (46, 246, 346, 446) such that the tensile force can be applied to the expandable tissue anchor (20, 120, 220, 320, 420) via the tether (52) and the elongate tension member (46, 246, 346, 446).

15. An anchor system (50) comprising the expandable tissue anchor (20, 120, 220, 320, 420) according to Claim 12, further comprising a second expandable tissue anchor, separate and distinct from the expandable tissue anchor (20, 120, 220, 320, 420).

16. The expandable tissue anchor (220, 320, 420) according to any one of Claims 1-5, 8-9, and 11-13 wherein the tissue-coupling portion (230, 330, 430) comprises a proximal portion (236, 336, 436) and a distal bullet head (222, 322, 422), which is fixed to a distal end of the proximal portion (236, 336, 436), and has, at a widest longitudinal site along the bullet head (222, 322, 422), a greatest outer cross-sectional area that equals at least 150% of an average outer cross-sectional area of the proximal portion (236, 336, 436).

## Patentansprüche

1. Expandierbarer Gewebeanker (20, 120, 190, 220, 320, 420), der konfiguriert ist, um in einer nicht expandierten, im Allgemeinen länglichen Konfiguration innerhalb eines Einsetzungswerkzeugs (70) an eine Herzkammer abgegeben zu werden, wobei der expandierbare Gewebeanker (20, 120, 190, 220, 320, 420) konfiguriert ist, um an einer Zielstelle an einer Herzgewebewand verankert zu werden, damit eine Zugkraft auf den expandierbaren Gewebeanker (20, 120, 190, 220, 320, 420) und somit auf die Herzgewebewand ausgeübt werden kann, sobald der expandierbare Gewebeanker (20, 120, 190, 220, 320, 420) eingesetzt ist, um so die Herzgewebewand an der Zielstelle relativ zum benachbarten Herzgewebe zu bewegen, wobei der expandierbare Gewebeanker (20, 120, 190, 220, 320, 420) Folgendes umfasst:
einen länglichen Gewebekopplungsteil (30, 130, 192, 230, 330, 430), der durch einen Ankerschaft (32, 194) an einem ersten Ende (34) des Gewebekopplungsteils (30, 130, 192, 230, 330, 430) gestützt wird, wobei der Gewebekopplungsteil (30, 130, 192, 230, 330, 430) konfiguriert ist, um in einer nicht expandierten, im Allgemeinen länglichen Konfiguration durch die Herzgewebewand von einer ersten Seite der Wand zu einer zweiten Seite der Wand abgegeben zu werden, wobei der Gewebekopplungsteil (30, 130, 192, 230, 330, 430) ferner konfiguriert ist, um sich auf der zweiten Seite der Herzgewebewand zu einer Konfiguration mit expandierter offener Spulenform im Allgemeinen orthogonal zum Ankerschaft (32, 194) zu expandieren, damit der expandierte Gewebekopplungsteil (30, 130, 192, 230, 330, 430) fest gegen die zweite Seite der Herzgewebewand an der Zielstelle gezogen werden kann, wenn die Zugkraft auf den Gewebekopplungsteil (30, 130, 192, 230, 330, 430) angewendet wird, wobei der Gewebekopplungsteil (30, 130, 192, 230, 330, 430) eine erste Steifheit aufweist; wobei er durch einen länglichen Spitzenteil (38, 138, 238, 338, 438) gekennzeichnet ist, der an einem zweiten Ende (40, 240, 340, 440) des Gewebekopplungsteils (30, 130, 192, 230, 330, 430) abgestützt ist, und konfiguriert ist, um durch die Herzgewebewand vorne vor dem Gewebekopplungsteil (30, 130, 192, 230, 330, 430) abgegeben zu werden, wobei der Spitzenteil (38, 138, 238, 338, 438) eine zweite Steifheit aufweist, die geringer ist als die erste Steifheit.

2. Expandierbarer Gewebeanker (20, 120, 190, 220, 320, 420) gemäß Anspruch 1, wobei der Spitzenteil (38, 138, 238, 338, 438) in Bezug auf den Gewebekopplungsteil (30, 130, 192, 230, 330, 430) axial fixiert ist.

3. Expandierbarer Gewebeanker (20, 120, 190, 320, 420) gemäß Anspruch 1, wobei der Spitzenteil (38, 138, 338, 438) nicht axial komprimierbar ist.

4. Expandierbarer Gewebeanker (20, 120, 190, 220, 320, 420) gemäß Anspruch 1, wobei die erste Steifheit des Gewebekopplungsteils (30, 130, 192, 230, 330, 430) mindestens 10 % größer ist als die zweite Steifheit des Spitzenteils (38, 138, 238, 338, 438).

5. Expandierbarer Gewebeanker (20, 120, 190, 220, 320, 420) gemäß Anspruch 1, wobei der Spitzenteil (38, 138, 238, 338, 438) eine Länge von mindestens 3 mm aufweist.

6. Expandierbarer Gewebeanker (20) gemäß Anspruch 1, wobei der Spitzenteil (38) eine Vielzahl von Schlitzen (42) aufweist, die die niedrigere zweite Steifheit des Spitzenteils (38) bereitstellen.

7. Expandierbarer Gewebeanker (120, 190) gemäß Anspruch 1, wobei ein durchschnittlicher Außendurchmesser des Spitzenteils (138) geringer ist als ein durchschnittlicher Außendurchmesser des Gewebekopplungsteils (130, 192), damit er die niedrigere zweite Steifheit des Spitzenteils (138) bereitstellt.

8. Expandierbarer Gewebeanker (20, 120, 190, 220, 320, 420) gemäß Anspruch 1, wobei der Spitzenteil (38, 138, 238, 338, 438) ein Material umfasst, das sich von dem des Gewebekopplungsteils (30, 130, 192, 230, 330, 430) unterscheidet, um so die niedrigere zweite Steifheit des Spitzenteils (38, 138, 238, 338, 438) relativ zur ersten Steifheit des Gewebekopplungsteils (30, 130, 192, 230, 330, 430) bereitzustellen.

9. Expandierbarer Gewebeanker (20, 420) gemäß einem der Ansprüche 1 bis 8, wobei der Gewebekopplungsteil (30, 430) und der Spitzenteil (38, 438) einstückig zueinander sind.

10. Expandierbarer Gewebeanker (20) gemäß Anspruch 9, wobei der Gewebekopplungsteil (30) und der Spitzenteil (38) eine hohle Röhre umfassen.

11. Expandierbarer Gewebeanker (320, 420) gemäß einem der Ansprüche 1 bis 5 oder 8 bis 9, wobei der Spitzenteil (338, 438) einen Kerndraht (356, 456) und einen Spulendraht (354, 454) umfasst, der um den Kerndraht (356, 456) gewickelt ist.

12. Expandierbarer Gewebeanker (20, 120, 220, 320, 420) gemäß einem der Ansprüche 1 bis 11, der ferner ein längliches Zugelement (46, 246, 346, 446) umfasst, das mit einem Teil des Gewebekopplungsteils (30, 130, 230, 330, 430) gekoppelt ist, damit die Zugkraft auf den Gewebekopplungsteil (30, 130, 230, 330, 430) angewendet werden kann, nachdem er zu der Konfiguration mit offener Spulenform expandiert wurde.

13. Expandierbarer Gewebeanker (220) gemäß Anspruch 12, wobei der Spitzenteil (238) eine einstückige distale Verlängerung des länglichen Zugelements (246) ist, und wobei der Spitzenteil (238) als geschlossene Schleife (244) mit einem distalen Ende (248) des länglichen Zugelements (246) geformt ist, das am Gewebekopplungsteil (230) nahe dem zweiten Ende (240) des Gewebekopplungsteils (230) fixiert ist.

14. Verankerungssystem (50), das den expandierbaren Gewebeanker (20, 120, 220, 320, 420) gemäß Anspruch 12 umfasst, wobei das Verankerungssystem (50) ferner ein am länglichen Zugelement (46, 246, 346, 446) befestigtes Halteband (52) umfasst, damit die Zugkraft über das Halteband (52) und das längliche Zugelement (46, 246, 346, 446) auf den expandierbaren Gewebeanker (20, 120, 220, 320, 420) angewendet werden kann.

15. Ankersystem (50), das den expandierbaren Gewebeanker (20, 120, 220, 320, 420) gemäß Anspruch 12 umfasst, ferner einen zweiten expandierbaren Gewebeanker umfasst, der von dem expandierbaren Gewebeanker (20, 120, 220, 320, 420) getrennt und verschieden ist.

16. Expandierbarer Gewebeanker (220, 320, 420) gemäß einem der Ansprüche 1 bis 5, 8 bis 9 und 11 bis 13, wobei der Gewebekopplungsteil (230, 330, 430) einen proximalen Teil (236, 336, 436) und einen distalen Kugelkopf (222, 322, 422) umfasst, welcher an einem distalen Ende des proximalen Teils (236, 336, 436) fixiert ist und an einer breitesten Längsseite entlang des Kugelkopfs (222, 322, 422) eine größte äußere Querschnittsfläche aufweist, die zumindest 150 % einer durchschnittlichen äußeren Querschnittsfläche des proximalen Teils (236, 336, 436) erreicht.

## Revendications

1. Ancrage de tissu extensible (20, 120, 190, 220, 320, 420) conçu pour être livré à une chambre cardiaque dans une configuration généralement allongée non-expansée à l'intérieur d'un outil de déploiement (70), l'ancrage de tissu extensible (20, 120, 190, 220, 320, 420) conçu pour être ancré à une paroi de tissu cardiaque au niveau d'un site cible de sorte qu'une force de traction puisse être appliquée sur l'ancrage de tissu extensible (20, 120, 190, 220, 320, 420) et ainsi sur la paroi de tissu cardiaque, une fois que l'ancrage de tissu extensible (20, 120, 190, 220, 320, 420) est déployé, de manière à déplacer la paroi de tissu cardiaque au niveau du site cible par rapport à un tissu cardiaque adjacent, l'ancrage de tissu extensible (20, 120, 190, 220, 320, 420) comprenant :
une partie allongée d'accouplement de tissu (30, 130, 192, 230, 330, 430) soutenue par un arbre d'ancrage (32, 194) au niveau d'une première extrémité (34) de la partie d'accouplement de tissu (30, 130, 192, 230, 330, 430), la partie d'accouplement de tissu (30, 130, 192, 230, 330, 430) conçue pour être livrée dans une configuration généralement allongée non-expansée à travers la paroi de tissu cardiaque à partir d'un premier côté de la paroi sur un second côté de la paroi, la partie d'accouplement de tissu (30, 130, 192, 230, 330, 430) étant en outre conçue pour s'étendre, sur le second côté de la paroi de tissu cardiaque, en une configuration de forme ouverte de bobine expansée généralement orthogonale sur l'arbre d'ancrage (32, 194), de sorte que la partie expansée d'accouplement de tissu (30, 130, 192, 230, 330, 430) puisse être fermement tirée contre le second côté de la paroi de tissu cardiaque au niveau du site cible lorsque la force de traction est appliquée sur la partie d'accouplement de tissu (30, 130, 192, 230, 330, 430), la partie d'accouplement de tissu (30, 130, 192, 230, 330, 430) ayant une première rigidité ; **caractérisé par** une partie de pointe allongée (38, 138, 238, 338, 438) soutenue au niveau d'une seconde extrémité (40, 240, 340, 440) de la partie d'accouplement de tissu (30, 130, 192, 230, 330, 430) et conçue pour être livrée à travers la paroi de tissu cardiaque avant la partie d'accouplement de tissu (30, 130, 192, 230, 330, 430), la partie de pointe (38, 138, 238, 338, 438) ayant une seconde rigidité inférieure à la première rigidité.

2. Ancrage de tissu extensible (20, 120, 190, 220, 320, 420) selon la revendication 1, dans lequel la partie de pointe (38, 138, 238, 338, 438) est fixée de manière axiale par rapport à la partie d'accouplement de tissu (30, 130, 192, 230, 330, 430).

3. Ancrage de tissu extensible (20, 120, 190, 320, 420) selon la revendication 1, dans lequel la partie de pointe (38, 138, 338, 438) n'est pas compressible de manière axiale.

4. Ancrage de tissu extensible (20, 120, 190, 220, 320, 420) selon la revendication 1, dans lequel la première rigidité de la partie d'accouplement de tissu (30, 130, 192, 230, 330, 430) est au moins 10 % supérieure à la seconde rigidité de la partie de pointe (38, 138, 238, 338, 438).

5. Ancrage de tissu extensible (20, 120, 190, 220, 320, 420) selon la revendication 1, dans lequel la partie de pointe (38, 138, 238, 338, 438) a une longueur d'au moins 3 mm.

6. Ancrage de tissu extensible (20) selon la revendication 1, dans lequel la partie de pointe (38) comprend une pluralité de fentes (42) qui fournit la seconde rigidité inférieure de la partie de pointe (38).

7. Ancrage de tissu extensible (120, 190) selon la revendication 1, dans lequel un diamètre externe moyen de la partie de pointe (138) est inférieur à un diamètre externe moyen de la partie d'accouplement de tissu (130, 192) de manière à fournir la seconde rigidité inférieure de la partie de pointe (138).

8. Ancrage de tissu extensible (20, 120, 190, 220, 320, 420) selon la revendication 1, dans lequel la partie de pointe (38, 138, 238, 338, 438) comprend un matériau différent de la partie d'accouplement de tissu (30, 130, 192, 230, 330, 430) de manière à fournir la seconde rigidité inférieure de la partie de pointe (38, 138, 238, 338, 438) par rapport à la première rigidité de la partie d'accouplement de tissu (30, 130, 192, 230, 330, 430).

9. Ancrage de tissu extensible (20, 420) selon l'une quelconque des revendications 1 à 8, dans lequel la partie d'accouplement de tissu (30, 430) et la partie de pointe (38, 438) sont intégrées l'une à l'autre.

10. Ancrage de tissu extensible (20) selon la revendication 9, dans lequel la partie d'accouplement de tissu (30) et la partie de pointe (38) comprennent un tube creux.

11. Ancrage de tissu extensible (320, 420) selon l'une quelconque des revendications 1 à 5 ou 8 à 9, dans lequel la partie de pointe (338, 438) comprend un fil de noyau (356, 456) et un fil à bobine (354, 454) enroulé autour du fil de noyau (356, 456).

12. Ancrage de tissu extensible (20, 120, 220, 320, 420) selon l'une quelconque des revendications 1 à 11, comprenant en outre un élément allongé de traction (46, 246, 346, 446) connecté à une partie de la partie d'accouplement de tissu (30, 130, 230, 330, 430) de sorte que la force de traction puisse être appliquée sur la partie d'accouplement de tissu (30, 130, 230, 330, 430) après avoir été élargie dans la configuration de forme ouverte de bobine.

13. Ancrage de tissu extensible (220) selon la revendication 12, dans lequel la partie de pointe (238) est une extension distale intégrale de l'élément allongé de traction (246), et dans lequel la partie de pointe (238) a la forme d'une boucle fermée (244), avec une extrémité distale (248) de l'élément allongé de traction (246) fixée à la partie d'accouplement de tissu (230) située à proximité de la seconde extrémité (240) de la partie d'accouplement de tissu (230).

14. Système d'ancrage (50) comprenant l'ancrage de tissu extensible (20, 120, 220, 320, 420) selon la revendication 12, dans lequel le système d'ancrage (50) comprend en outre un câble d'attache (52) fixé à l'élément allongé de traction (46, 246, 346, 446) de sorte que la force de traction puisse être appliquée sur l'ancrage de tissu extensible (20, 120, 220, 320, 420) par l'intermédiaire du câble d'attache (52) et de l'élément allongé de traction (46, 246, 346, 446).

15. Système d'ancrage (50) comprenant l'ancrage de tissu extensible (20, 120, 220, 320, 420) selon la revendication 12, comprenant en outre un second ancrage de tissu extensible, séparé et distinct de l'ancrage de tissu extensible (20, 120, 220, 320, 420).

16. Ancrage de tissu extensible (220, 320, 420) selon l'une quelconque des revendications 1 à 5, 8 à 9, et 11 à 13, dans lequel la partie d'accouplement de tissu (230, 330, 430) comprend une partie proximale (236, 336, 436) et une tête de boulon distale (222, 322, 422), qui est fixée à une extrémité distale de la partie proximale (236, 336, 436), et a, au niveau d'un site longitudinal le plus large le long de la tête de boulon (222, 322, 422), une zone transversale externe la plus importante qui représente au moins 150 % d'une zone transversale externe moyenne de la partie proximale (236, 336, 436).
